# EUROPÄISCHE PATENTSCHRIFT

(11) **EP 2 091 537 B1**
(45) Veröffentlichungstag und Bekanntmachung des Hinweises auf die Patenterteilung: **22.05.2013**
(21) Anmeldenummer: 07846885.7
(22) Anmeldetag: 23.11.2007
(51) Int. Cl.: A61K 31/48, A61K 31/00, A61P 9/10, A61P 9/08

(54) **PHARMAZEUTISCHE ZUSAMMENSETZUNGEN ZUR BEHANDLUNG VON KAPILLARER ARTERIOPATHIE**
PHARMACEUTICAL COMPOSITIONS FOR THE TREATMENT OF CAPILLARY ARTERIOPATHY
COMPOSITIONS PHARMACEUTIQUES POUR LE TRAITEMENT DE L'ARTÉRIOPATHIE CAPILLAIRE

(30) Priorität: 23.11.2006 EP 06024308; 06.12.2006 EP 06025263
(43) Veröffentlichungstag der Anmeldung: 26.08.2009
(73) Patentinhaber: Sinoxa Pharma GmbH, 14195 Berlin (DE)
(72) Erfinder: REITER, Rudolf, 6940 Appenzell (CH); TACK, Johannes, 13595 Berlin (DE); HOROWSKI, Reinhard, 13353 Berlin (DE)
(74) Vertreter: Kilger, Ute
(86) Internationale Anmeldenummer: PCT/EP2007/010360
(87) Internationale Veröffentlichungsnummer: WO 2008/061805

(56) Entgegenhaltungen:
- WO-A-2005/049088
- WO-A1-02/094238
- DE-A1- 4 240 798
- GB-A- 2 192 541
- US-A- 4 593 032
- US-A1- 2002 122 773
- US-A1- 2006 105 030
- SETOLA V ET AL: "3,4-Methylenedioxymethamphetamine (MDMA, "Ecstasy") induces fenfluramine-like proliferative actions on human cardiac valvular interstitial cells in vitro" MOLECULAR PHARMACOLOGY 01 JUN 2003 UNITED STATES, Bd. 63, Nr. 6, 1. Juni 2003 (2003-06-01), Seiten 1223-1229, XP002469125 ISSN: 0026-895X
- JAEHNICHEN SVEN ET AL: "Agonism at 5-HT2B receptors is not a class effect of the ergolines" EUROPEAN JOURNAL OF PHARMACOLOGY, Bd. 513, Nr. 3, April 2005 (2005-04), Seiten 225-228, XP002469123 ISSN: 0014-2999
- REILLY A ET AL: "Raynaud Phenomenon: Whether it's primary or secondary, there is no cure, but treatment can alleviate symptoms." AJN, AMERICAN JOURNAL OF NURSING, [Online] Bd. 105, Nr. 8, August 2005 (2005-08), Seiten 56-65, XP002469124 Gefunden im Internet: URL:http://www.scleroderma.org/pdf/Medical Articles/2006/RaynaudsPhenomenon.pdf> [gefunden am 2005-08-01]
- HACHULLA ET AL: "Sclerodermie systemique" EMC - RHUMATOLOGIE-ORTHOPEDIE, EDITIONS SCIENTIFIQUES ET MEDICALES ELSEVIER, Bd. 2, Nr. 5, September 2005 (2005-09), Seiten 479-500, XP005097354 ISSN: 1762-4207
- OBESO J A ET AL: "Intravenous lisuride corrects oscillations of motor performance in Parkinson's disease", ANNALS OF NEUROLOGY 1986 US, vol. 19, no. 1, 1986, pages 31-35, ISSN: 0364-5134
- OBESO J A ET AL: "Intravenous lisuride corrects oscillations of motor performance in Parkinson's disease", 1986, ANNALS OF NEUROLOGY 1986 US, VOL. 19, NR. 1, PAGE(S) 31 - 35 ISSN: 0364-5134
- LUQUIN M R ET AL: "Parenteral administration of lisuride in Parkinson's disease.", ADVANCES IN NEUROLOGY 1987 LNKD- PUBMED:3825733, vol. 45, 1987, pages 561-568, XP001525514, ISSN: 0091-3952

## Beschreibung

Die vorliegende Erfindung betrifft die Verwendung von spezifischen Ergot-Derivaten bzw. Ergotinen. Insbesondere von Lisurid und Tergurid zur Prophylaxe und Behandlung von konstriktiver kapillärer Arteriophatie. Mit konstriktiver kapillärer Arteriophatie werden die Erkrankungen pulmonaler arterieller Hochdruck:, endogen-induzierte oder exogen-induzierte Glomerulosklerosen sowie sekundäres Raynaud Syndrom bzw. Phänomen bezeichnet.

Konstriktive kapilläre Arteriophatie ist ein pathologisches Kennzeichen in der Humanmedizin für diffuse konstriktive arterielle Läsionen umfassend eine Umgestaltung der Gefäßwand, welche zu irreversiblen Verengungen bis hinzu Verschlüssen von Arteriolen führt. Als funktionale Konsequenzen ist ein Anstieg des Gefäßdrucks und ein erhöhter vaskulärer Wiederstand zu beobachten.

Die konstriktive kapillare Arteriopathie verschiedener Ätiologie manifestiert sich in dem Gefäßbett vieler Gewebearten. Die vorliegende Erfindung fokussiert sich im Rahmen der kapillaren Arteriopathie auf organspezifische Veränderungen, welche zum Anstieg des Langzeitdrucks von Arteriolen führen und sich durch die Erhöhung des vaskularen Widerstandes, aggrevativer Vasospasmus und prezipitierendem strukturellem Verschluss auszeichnen. Somit steht der Begriff "kapillare Arteriopathie" und insbesondere "konstriktive kapillare Arteriopathie" wie hierin verwendet für die Indikationen arterieller Lungenhochdruck, Glomerulosklerosen und sekundäres Raynaud Phänomen und/oder Syndrom.

Aufgabe der vorliegenden Erfindung ist es, weitere Verwendungen von von Lisurid und Tergurid bereitzustellen.

Die Aufgabe wird durch die in Patentanspruch 1 beschriebenen Indikationen gelöst. Weitere vorteilhafte Ausgestaltungen ergeben sich aus den abhängigen Ansprüchen, den Beispielen und der Beschreibung.

Überraschenderweise wurde festgestellt, dass die beanspruchten Ergot-Derivate und Ergoline und insbesondere Lisurid und Tergurid für die Prophylaxe als auch die Behandlung von (konstriktiver) kapillärer Arteriophatie geeignet sind.

Die vorliegende Erfindung betrifft somit die Verwendung von Lisurid und Tergurid

Salze, Enantiomere, Enantiomerengemische, Diastereomere, Diastereomerengemische, Hydrate, Solvate und Racemate der vorgenannten Verbindungen zur Herstellung einer pharmazeutischen Zusammensetzung zur Behandlung und Prophylaxe von konstriktiver kapillärer Arteriophatie, d.h. von pulmonaler arterieller Hypertension, endogen-induzierter oder exogen-induzierter Glomerulosklerosen sowie von sekundärem Raynaud Syndrom.

Lisurid und Tergurid sind basisch und durch Zugabe von organischen oder anorganischen Säuren können Säureadditionssalze erhalten werden. Als Säuren, welche ein Säureadditionssatz mit Lisurid oder Tergurid bilden, können die folgenden genannt werden: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-Mandelsäure, Hydrogenbenzolsulfonsäure, Pikrinsäure (2,4,6-Trinitrophenol), Adipinsäure, d-o-Tolylweinsäure, Aminosäuren wie Methionin, Tryptophan, Arginin und insbesondere saure Aminosäuren wie Glutaminsäure oder Asparaginsäure.

Bei dem Vorhandensein von sauren Gruppen lassen sich auch Basenadditionssalze bilden, z.B. Alkalimetallsalze sowie Salze mit Aminen. So können Alkalimetallsalze wie das Natriumsalz, das Kaliumsalz, das Lithiumsalz oder das Magnesiumsalz, das Calciumsalz, Alkylaminosalze oder Aminosäurensalze, z.B. mit basischen Aminosäuren wie Lysin gebildet werden.

Die beanspruchten Ergot-Derivate umfassen auch Stereoisomere, Enantiomere, Enantiomerengemische, Diastereomere und Diastereomerengemische, wobei beispielsweise chirale Verbindungen bevorzugt

Insbesondere bevorzugt sind Lisurid (CAS-Nr.: 18016-80-3, 3-(9,10-Didehydro-6-methylergolin-8alpha-yl)-1,1-diethylharnstoff), und Tergurid ((+)-1,1-Diethyl-3-(6-methyl-8a-ergolinyl)-harnstoff). Im speziellen bevorzugt ist die Verwendung von Tergurid (trans-Dihydrolisurid) und Lisurid.

Die vorgenannten Substanzen eignen sich insbesondere zur Prophylaxe und Behandlung von pulmonalem arteriellem Hochdruck, endogen-induzierten oder exogen-induzierten Glomerulosklerosen sowie sekundäres Raynaud Phänomen bzw. Raynaud Syndrom.

Dass sich die beanspruchten Verbindungen zur Prophylaxe und Behandlung von konstriktiver kapillärer Arteriophatie eignen, war überraschend, da ein Fachmann aufgrund des Standes der Technik derartige Verbindungen nicht in Betracht gezogen hätte, da gerade diese Indikation als Nebenwirkungen bei bei 8-α-Ergolinen genannt werden. So beschreibt die Firma Schering AG in ihrem Beipackzettel zum Arzneimittel Teluron^{®}, welches als 8-α-Ergolin Tergurid enthält, dass beispielsweise Raynaud Phänomen bzw. Raynaud Syndrom auftreten kann. Ferner ist literaturbekannt, dass Mutterkornalkaloide, welche auch die 8-α-Ergoline umfassen, fibrotische Veränderungen zur Folge haben können. Explizit als Nebenwirkungen sind Raynaud Phänomen, Vasospasmus, Diplopie, retroperitoneale Fibrose, Pleuraergüsse und Herzklappenfibrosen dem Fachmann bekannt. Diese Negativbefunde haben einen Fachmann davon abgehalten, die Verbindungen der Formel (I) zur Prophylaxe und Behandlung der vorgenannten Indikationen einzusetzen.

Somit würde ein Fachmann die beanspruchten Verbindungen insbesondere Lisurid und Tergurid nicht zur Prophylaxe und Behandlung von konstriktiver kapillarer Arteriophatie gekennzeichnet durch die Erkrankungen pulmonalem arteriellem Hochdruck, endogen-induzierten oder exogen-induzierten Glomerulosklerosen sowie sekundäres Raynaud Syndrom einsetzen. Insbesondere kommen für einen Fachmann die Indikationen Raynaud Phänomen bzw. Raynaud Syndrom und pulmonale Hypertension nicht in Betracht, da diese explizit als Nebenwirkungen für 8-α-Ergolin-Wirkstoffen genannt werden. Gerade die Erwähnung derartiger Nebenwirkungen in einem Beipackzettel für ein Pharmazeutikum aus der Wirkstoffklasse der 8-α-Ergoline weist einen Fachmann konkret darauf hin, dass eine intensive klinische Untersuchung des Wirkstoffs stattgefunden hat. Somit besteht kein Anlaß für einen Fachmann an den Angaben und Aussagen in einem Beipackzettel eines Pharmazeutikums zu zweifeln.

Posturale Hypertension ist als eine Nebenwirkung von dopaminergen Ergotderivaten einschließlich Lisurid und Tergurid bekannt. Da die Verabreichung von Ergolinen und Ergotderivaten mit stärkeren gastrointestinalen Nebenwirkungen wie beispielsweise Brechreiz und Übelkeit verbunden ist, wurde ihnen ein therapeutischer Nutzen teilweise grundsätzlich abgesprochen.

Um so überraschender war es als festgestellt wurde, dass Tergurid und Lisurid gerade bei den Erkrankungen pulmonale arterielle Hypertension (PAH), endogen-induzierte oder exogen-induzierte Glomerulosklerosen sowie sekundäres Raynaud Phänomen bzw. Syndrom, welche hier zusammen als konstriktive kapilläre Arteriophatie bezeichnet werden, eine therapeutische Wirkung zeigen und eben nicht wie zu erwarten war, kontraindikativ sind.

Hypertension ist der medizinische Fachausdruck für hohen Blutdruck. Der Begriff "Blutdruck" bezeichnet den Druck, der entsteht, wenn Blut entlang der inneren Gefäßwand der Arterien zirkuliert. Der Blutdruck wird in der Regel durch zwei Werte angegeben, nämlich den arteriellen Druck, wenn das Herz sich zwischen den einzelnen Herzsschlägen kontrahiert und wieder relaxiert (d.h. den systolischen und den diastolischen Druck).

Der Blutdruck verändert sich normalerweise über den Tag hinweg und nimmt normalerweise mit steigendem Alter zu. Zusätzlich beeinflussen körperliche Aktivitäten den Blutdruck. Der Blutdruck steigt als Reaktion auf körperliche Belastung und Stress. Patienten mit Hypertension haben einen erhöhten Blutdruck (meist über 140/90 mm Hg) auch im Ruhezustand. Unbehandelte Hypertension führt dazu, dass das Herz und auch die Arterien einer stärkeren Belastung ausgesetzt sind, was zu einer Schädigung des Gewebes führen kann. Dies ist wiederum ein Risikofaktor und kann zu Herzfehlern, Herzinfarkt (Myokardinfarkt) und Schlaganfall führen.

Im Gegensatz dazu führen beispielsweise die pulmonale Hypertension oder die Glomerulosklerosen zu einer lokalen Veränderung der Vasoreaktivität, was zu einem lokalen Anstieg des Blutdrucks führt, ohne einen detektierbaren Anstieg des systemischen Blutdrucks hervorzurufen.

Beispielsweise äußert sich bei pulmonaler Hypertension der hohe Blutdruck nur in der pulmonalen Zirkulation. Hingegen ist der Blutdruck in beispielsweise den Armen oder im Rest des Körpers normal und niedriger. Daher ist pulmonale Hypertension deutlich verschieden von (allgemeiner) Hypertension. Pulmonale Hypertension ist in der Regel die Folge einer Erkrankung des Herzens und/oder der Lungen. Pulmonale Hypertension liegt vor, wenn der Blutdruck in den pulmonalen Arterien den normalen systemischen Blutdruck übersteigt, was auf lokale Veränderungen der Vasoreaktivität und Aufbau der kleinen Arterien, den sogenannten Arteriolen zurückzuführen ist. Dies führt zu einer Belastung der rechten Herzseite. Pulmonale Hypertension ist ein ernsthaftes Problem. Sie äußert sich in Anzeichen wie Kurzatmigkeit nach geringer Anstrengung, Müdigkeitsgefühl, Ohnmacht und Brustschmerzen. Diese Symptome beschränken gewöhnlich körperliche Belastungen und Aktivitäten.

Der Unterschied in der Ätiologie sowie unterschiedliche Behandlungsansätze für Hypertension (auch bezeichnet als essentielle oder allgemeine Hypertension) und pulmonale Hypertension lassen den deutlichen Unterschied zwischen diesen beiden Krankheiten erkennen. Während ACE-Inhibitoren wie z.B. Captopril, Diuretika wie z.B. Furosemid, Angiotensin-2-Rezeptorblocker wie Z.B. Losartan, alpha- und beta-Blocker wie z.B. Prazosin und Propranolol, direkte Vasodilatoren wie z.B. Minoxidil oder zentral wirkende Agentien wie z.B. Clonidin zur Behandlung von Hypertension eingesetzt werden, eignet sich keiner dieser Wirkstoff für die spezifische Behandlung von pulmonaler Hypertension und wird auch nicht dafür verwendet.

Somit kann aus einer positiven Wirkung von insbesondere Tergurid und Lisurid bei pulmonalem arteriellem Hochdruck nicht auch gleichzeitig eine positive Wirkung bei Hypertension abgeleitet werden oder umgekehrt.

Ferner betrifft die vorliegende Erfindung pharmazeutische Zusammensetzungen, welche unter Verwendung mindestens einer beanspruchten Verbindung oder eines Salzes davon und insbesondere unter Verwendung von Lisurid oder Tergurid hergestellt wurden.

Diese pharmazeutischen Zusammensetzungen enthalten mindestens eine Verbindung gemäß Anspruch 1 und insbesondere Lisurid oder Tergurid in einer aktiven Wirkstoffkonzentration von 0,1 - 10 mg pro Einzeldosis zusammen mit mindestens einem pharmakologisch verträglichen Träger, Hilfsstoff oder Lösungsmittel.

Die pharmazeutischen Zusammensetzung werden bevorzugt in Form von Tabletten, Schichttabletten, Pillen, Kapseln, Mikrokapseln, Retard-Oralia, transdermalen Systemen, Suppositorien, Mikroformulierungen, Nanoformulierungen, liposomalen Formulierungen, Tropfen, Nasentropfen, Nasensprays, Aerosolen, Ampullen, Lösungen, Emulsionen, Dispersionen, Pulvern, Inhalationspulvern, mikrokristallinen Formulierungen oder Inhalationssprays bereit gestellt und sind insbesondere zur oralen, sublingualen, parenteralen, kutanen, bukkalen, perkutanen, inhalativen oder nasalen Verabreichung geeignet.

Als pharmakologisch verträgliche Träger können beispielsweise Lactose, Stärke, Sorbitol, Sucrose, Cellulose, Magnesiumstearat, Dicalciumphosphat, Calciumsulfat, Talk, Mannitol, Ethylalkohol und dergleichen eingesetzt werden. Puder als auch Tabletten können zu 5 bis 95% aus einem derartigen Träger bestehen.

Als Bindemittel können zudem Stärke, Gelatine, natürliche Zucker, sowohl natürliche als auch synthetische Gummis wie beispielsweise Akaziengummi oder Guar-Gummi, Natriumalginat, Carboxymethyl-Cellulose, Polyethylenglycol und Wachse eingesetzt werden. Als Gleitmittel können Borsäure, Natriumbenzoat, Natriumacetat, Natriumchlorid, und dergleichen dienen.

Ferner können den pharmazeutischen Zusammensetzungen noch Sprengmittel, Farbstoffe, Geschmacksstoffe und/oder Bindemittel zugesetzt werden.

Flüssige Formulierungen umfassen Lösungen, Suspensionen, Sprays und Emulsionen. Beispielsweise Injektionslösungen auf Wasserbasis oder Wasser-Propylenglycol-Basis für parenterale Injektionen.

Für die Zubereitung von Suppositorien werden bevorzugt niedrigschmelzende Wachse, Fettsäureester und Glyceride eingesetzt.

Kapseln werden beispielsweise aus Methylcellulose, Polyvinylalkohole oder denaturierte Gelatine oder Stärke hergestellt.

Als Sprengmittel können Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, Natrium-Carboxymethylcellulose, microkristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Korn, Reis oder Kartoffeln, natürliche Gummis wie Akaziengummi, Gelatine, Tragacanth, Alginsäure, Natriumalginat, Ammonium-Calcium-Alginat, Methylcellulose, Natrium-Carboxymethylcellulose, Hydroxypropyl-methylcellulose, Polyvinylpyrrolidon sowie anorganische Verbindungen wie Magnesium-Aluminum-Silicate zugesetzt werden. Die Bindemittel könne in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol und Aminosäuren wie Leucin eingesetzt werden. Derartige Gleitmittel können in Mengen von 0,05 bis 15 Gew.-% verwendet werden.

Als weitere bevorzugte Formulierungen sind subkutane Formulierungen und transdermale Systeme zu nennen. Solche subkutanen Formulierungen und Transdermalsysteme bestehen vorzugsweise aus einer Matrix insbesondere eine bioabbaubare polymere Matrix, worin die mindestens eine Verbindung gemäß Formel (I) bevorzugt Lisurid oder Tergurid eingelagert ist. Zur Erzeugung dieser Matrix werden vorzugsweise biologisch abbaubare Polymere verwendet.

Als Beispiele für bioabbaubare Polymere können genannt werden: Polyvalerolactone, Poly-ε-Decalactone, Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäureanhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherester-multiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonate, Polytrimethylcarbonate, Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan] Polyhydroxypentan-säure, Polyanhydride, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Heparansulfat, Heparin, Chondroitinsulfat, Dextran, b-Cyclodextrine, und Copolymere mit PEG und Polypropylenglycol, Gummi arabicum, Guar, Gelatine, Collagen Collagen-N-Hydroxysuccinimid, Modifikationen und Copolymere und/oder Mischungen der vorgenannten Substanzen.

Bevorzugt sind biologische Polymere wie beispielsweise Stärke und denaturierte Stärke, Cellulose, Glykosaminoglykane und Collagen als auch semisynthetische und synthetische Polymere wie beispielsweise Silicone, Siliconelastomere, Polydimethylsiloxan, Polydimethylsiloxan enthaltend Siliciumdioxid, Polydimethylsiloxan enthaltend Polyalkylenoxid (Gelest^{®}), Polytetrafluorethylen (Teflon^{®}), Polylactide, Polyglykolide, Polyethylenglykol, Polylactid-Polyglykolid-Copolymere, Polyanhydride, Ethylenvinylacetat-Polymere, Poly(methylmethacrylat), Celluloseethylether, Poly(ethylacrylat), Poly(trimethylammoniumethyl-methacrylate), Polydimethylsiloxane, Hydroxyethyl-Polymethacrylate, Polyurethane und Polystyrol-Butadien-Copolymere.

Ferner können derartige transdermale Systeme auch aus mikrosphärischen Partikeln oder Nanopartikeln oder Mikrokristallen bestehen, welche mindestens eine Verbindung gemäß allgemeiner Formel (I) enthalten. Solche Partikel können zudem in ein Gel eingebracht und in dieser Form appliziert werden.

Ferner ist auch die Verwendung von Mikropartikeln aus biokompatiblen Keramiken wie beispielsweise Hydroxyapatit möglich, an und/oder in welche die Verbindungen gemäß Formel (I) angelagert und/oder eingelagert werden.

### Figurenbeschreibung

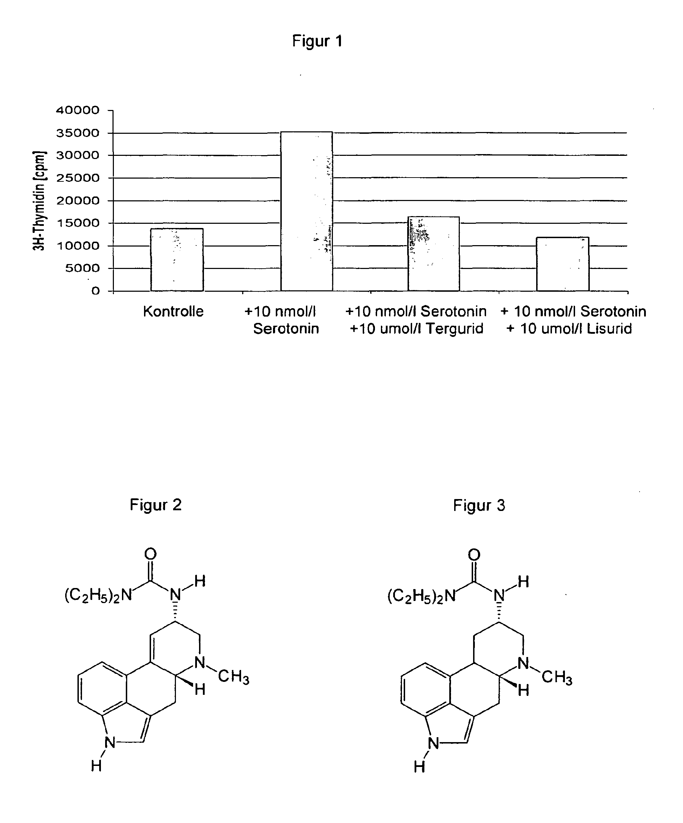

- Figur 1: zeigt, dass in Gegenwart von erhöhten Serotonin-Konzentrationen, Serotonin als Wachstumsfaktor zu einer Proliferation von glatten Muskelzellen führt. In Anwesenheit einer Verbindung gemäss allgemeiner Formel (I) wie beispielsweise Lisurid oder Tergurid wird diese Zellproliferation durch die antagonistische Wirkung der Substanzen (an Serotonin 5-HT2 Rezeptoren) deutlich vermindert. Aus diesem in vitro Modell kann abgeleitet werden, dass unter Bedingungen, die lokal oder systemisch zu einer erhöhten Serotoninfreisetzung führen, die genannten Stoffe eine überschiessende Proliferation von glatten Muskelzellen in Blutgefäßen während des Heilungsprozesses hemmen können.
- Figur 2: zeigt die chemische Struktur von Lisurid,
- Figur 3: zeigt die chemische Struktur von Tergurid.

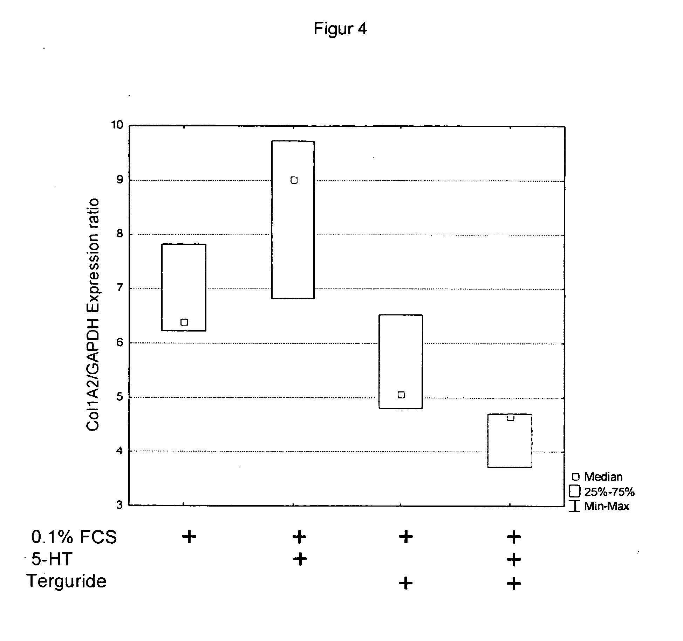

- Figur 4: zeigt den Einfluß von Serotonin und Tergurid auf die Expression von Col1 A2 in glatten Muskelzellen aus Lungenarterien.

### Beispiele

### Beispiel 1: antiproliferative Wirkung

Humane pulmonale glatte Muskelzellen (PromoCell) wurden in PromoCell-Kulturmedium nach Angaben des Herstellers bis zur Konfluenz in Sechs-LochPlatten kultiviert. Die pulmonalen glatten Muskelzellen wurden danach in PromoCell-Kulturmedium in 24-Loch-Plattenin einer Zelldichte von 5 * 10⁴ Zellen pro Loch (Well) ausgesät. Nach erfolgter Adhäsion der Zellen wurde das Kulturmedium ausgetauscht und durch Kultur in einem Medium mit 0,2% fötalem Kälberserum über 48 Stunden ein Wachstumsarrest herbeigeführt.

Zur Prüfung der antiproliferativen Substanzwirkung wurden die Zellen zunächst mit 10 µmol/l Wirkstoffe vorinkubiert. Daraufhin wurde das Wachstumsverhalten der Zellen mit Serotonin (10⁻⁸ mol/l) stimuliert. Zur Messung der Zellproliferation wurden [3H]-Thymidin (Amersham) zu den Kulturen zugesetzt und für 24 Stunden inkubiert. Danach wurden die Zellen zunächst zweimal in eiskalter Phosphat-gepufferter Kochsalzlösung und anschließend in eiskalter 10%iger Trichloressigsäure für 30 Minuten bei 4°C inkubiert. Die Zellen wurden dann in 0,1 molarer Natronlauge (0,5 ml/Loch) aufgelöst. Nach Neutralisation mit Essigsäure wurde der Einbau von [3H]-Thymidin durch Flüssigkeitsszintillationsmessungen bestimmt. Die Bestimmungen wurden als Triplikate ausgeführt. In Figur 1 ist jeweils der Mittelwert dargestellt.

### Beispiel 2: Herstellung einer Formulierung zur oralen Anwendung mit Tergurid.

25,0 g Tergurid, mikronisiert werden mit 4035,0 g Laktose, 1800,0 g mikrokristalliner Cellulose und 120,0 g Croscarmellose Na nach vorherigem Sieben der Hilfsstoffe für 5 min. in einem Freifallmischer bei z. Bsp. 162 rpm gemischt. Anschliessend wird diese Vormischung über ein Sieb mit 0,8 mm Maschenweite gegeben. Es wird 20,0 g Magnesiumstearat zugegeben und erneut für 1 min. gemischt. Die so erhaltene Pressmasse wird auf einer geeigneten Tablettenpresse (z. Bsp. Rundläuferpresse) zu 50.000 Tabletten (theoretische Ausbeute) mit 7mm Durchmesser und einem Tablettengewicht von 120 mg entsprechend einer Dosierung von 0,5 mg Tergurid /Tablette verpresst. Die so hergestellten Tabletten geben den Wirkstoff nach Einbringen in Wasser schnell und nach max. 60 min. nahezu vollständig frei.

### Beispiel 3: Herstellung einer Formulierung zur oralen Anwendung von Lisurid mit verzögerter Freisetzung.

2,0 g Lisurid Hydrogenmaleat, mikronisiert werden mit 750,0 g Hydroxyethylcellulose (Tylose H) und 243,0 g mikrokristalliner Cellulose nach vorherigem Sieben der Hilfsstoffe für 5 min. in einem Freifallmischer bei z. Bsp. 180 rpm gemischt. Anschliessend wird diese Vormischung über ein Sieb mit 0,8 mm Maschenweite gegeben. Es wird 5,0 g Magnesiumstearat zugegeben und erneut für 1 min. gemischt. Die so erhaltene Pressmasse wird auf einer geeigneten Tablettenpresse (z. Bsp. Rundläuferpresse) zu 10.000 Tabletten (theoretische Ausbeute) mit 6 mm Durchmesser und einem Tablettengewicht von 100 mg entsprechend einer Dosierung von 0,2 mg Lisurid Hydrogenmaleat / Tablette verpresst. Die so hergestellten Tabletten geben den Wirkstoff nach Einbringen in Wasser verzögert frei, sodass nach ca. 2 h etwa 60 - 70% der Dosis aus der Formulierung abgegeben werden.

### Beispiel 4: Herstellung eines sterilen Lyophilisates mit Lisurid Hydrogenmaleat zur Injektion nach Auflösung.

2,0 g Lisurid Hydrogenmaleat werden zusammen mit 20,0 g Laktose-Monohydrat, 0,4 g Citronensäure-Monohydrat und 1,0 g Natriumcitrat-Dihydrat in 976,6 g Wasser für Injektionszwecke gelöst. Die erhaltene farblos bis schwach gelbliche Lösung weist einen pH Wert zwischen 4,5 und 5,4 auf. Diese Lösung über ein Membranfilter vorfiltriert und danach über einen weiteren Membranfilter (0,2 µm) unter aseptischen Bedingungen sterilfiltriert. Je 1,0 g der so erhaltenen sterilen Lösung wird in sterilisierte Vials mit einem Füllvolumen von 6 ml verfüllt mit einem für die anschliessende Gefriertrocknung geeigneten Gummistopfen versehen und in einem Lyophilisator auf -40 bis -50°C eingefroren. Anschliessend wird unter Vakuum getrocknet bzw. nachgetrocknet unter Erhalt eines trockenen Substanzkuchens. Die Vials werden unter aseptischen Bedingungen verschlossen und gebördelt. Es werden auf diesem Wege 1000 Vials (theoretische Ausbeute) mit je 2 mg lyophilisiertem Lisurid Hydogenmaleat. Das Lyophilisat wird durch auslösen mit steriler, physiologischer Kochsalz Lösung rekonstituiert und ergibt eine gebrauchsfertige sterile Lösung zur Injektion bzw. Infusion zur sofortigen Anwendung.

### Beispiel 5: Herstellung eines Matrixpflasters mit Tergurid zur transdermalen Anwendung

2,5 g Tergurid werden mit 2,13 g Aceton und 51,54 g einer Lösung von basischem Butylmethacrylate Copolymer (Eudragit 100 Lösung) zusammengegeben. Der Lösung werden 5,0 g Polyvinylpyrrolidone (Povidone 25), 2,5 g Propylenglycol, 5,0 g Dodecyl-N,N-dimethylaminoacetat (alternativ 5,0 g 1-Dodecanol), 1,0 g Foral E 105 und 0,65 g eines Antioxidants (z. Bsp. Butylhydroxyanisol) zugegeben. Die so erhaltene Beschichtungslösung wird unter geeigneten Prozessbedingungen in einem Coater auf eine Polymerfolie aus Polyethylen kontinuierlich ausgestrichen und anschliessend getrocknet zu einem Flächengewicht von 50 mg/10cm² (± 5%) beschichteter Fläche. Die so erhaltene klebende Matrix wird mit einer einseitig silikonisierten Polymerfolie laminiert und in einem weiteren Schritt zu Pflastern in einer für die therapeutische Anwendung geeigneten Größe (z. Bsp. 20 cm²) gestanzt und in Alubeuteln verpackt. Das so hergestellte Tergurid Pflaster gibt den Wirkstoff nach Applikation auf die intakte unbehaarte Haut kontinuierlich über mehrere Tage mit einer Geschwindigkeit zwischen 0,1 bis 0,5 µg/cm²/h an die systemische Zirkulation ab.

### Beispiel 6: Herstellung eines Membranpflasters mit Lisurid zur transdermalen Anwendung.

Mittels eines Laborcoaters wird eine Membran aus mikroporösem Polyethylen (Solupor® 10P05A) als Kontrollmembran (oder alternativ aus Ethylenvinylacetat Copolymer (EVA, Cotran® 3M 9728)) mit einem hautverträglichen Siliconkleber (BioPSA®7- 4202) (alternativ Polyisobutylenkleber, Oppanol^{®}), beschichtet und getrocknet mit einem Flächengewicht von ca. 10 bis 25 mg/cm² und anschliessend mit einem einseitig silikonisierten Releaseliner (Polyethylen) laminiert.

Das so erhaltene Laminat wird in einer geeigneten Siegelmaschine mit heisssiegelfähigem Polyethylen ringförmig bis auf eine kleine Öffnung gesiegelt und gestanzt. In den entstandenen Hohlraum wird über die verbleibende Öffnung mittel einer geeigneten Injektionsvorrichtung ca. 0,5 ml einer 1%igen Lösung von Lisurid in 2-Propanol, Hydroxypropylcellulose (Klucel^{®} LF) und Tocopherol eingegeben und danach vollständig versiegelt.

Nach Äquilibierung und Abzug des Releaseliners kann das Membranpflaster auf die intakte, unbehaarte Haut aufgeklebt werden und gibt kontinuierlich und mit konstanter Geschwindigkeit Lisurid frei. Die Dosierung kann durch die unterschiedliche Pflastergrößen eingestellt werden.

### Beispiel 7: Herstellung einer sterilen, subcutan zu applizierenden Formulierung mit Tergurid

50 g Tergurid mikronisiert werden mit 50 g Polydimethylsiloxane homogen vermischt und mit Standardverfahren vorzugsweise durch Extrusion zu einer strangförmigen Core-Matrix geformt. Der Strang wird in Teile zu 30 mm geschnitten. Nach dem gleichen Verfahren wird ein wirkstofffreies Core-Extrudat mit identischen Dimensionen hergestellt. Aus käuflich erhältlichem Siliciumdioxid enthaltendem Polydimethylsiloxan oder aus z. Bsp. Pt katalysiertem vernetztem Polyalkylenoxid (Gelest®) enthaltendem Polydimethylsiloxan werden in einem zweiten Schritt durch Extrusion schlauchförmige Membranen mit einer Wandstärke von z. Bsp. 0,2 mm Wandstärke hergestellt. Die Membranen werden zu einer Länge von 60 mm geschnitten und in Cyclohexan schwellen lassen. Anschliessend wird die wirkstoffhaltige Core Matrix eingeführt und von beiden Seiten der Schlauchmembran das wirkstofffreie Extrudat eingeführt, z. Bsp. in einer Weise, dass auf beiden Seiten ein luftgefüllter Zwischenraum von ca. 1-3 mm zwischen dem wirkstoffhaltigen Core und dem wirkstofffreien Core entsteht. Anschliessend wird Cyclohexan durch Verdunsten entfernt, die Formulierung auf eine Gesamtlänge von 50 mm geschnitten, sodass an beiden Seiten der Formulierung ein Verschluss durch wirkstofffreies Corematerial entsteht. Die Formulierung wird durch Standardverfahren (Ethylenoxid, H₂O₂) gassterilisiert. Durch die Lufteinschlüsse kann die Lage der Formulierung am Applikationsort durch Ultraschalldetektion jederzeit detektiert werden.

### Beispiel 8: Pulmonaler Hochdruck:

### Versuchsbeschreibung:

Ratten wurden am Versuchstag **1** Monocrotalin (60 mg/kg; Sigma) verabreicht. Die Substanz wurde hierzu in 0.5 molarer Salzsäure gelöst und anschliessend der pH mit 0.5 molarer Natronlauge auf 7.4 adjustiert. Die Lösung wurde als einmalige subkutane Injektion in einer Dosis von 60 mg/kg an männliche Sprague Dawley Ratten verabreicht. Kontrolltieren wurde das gleiche Volumen isotonischer Kochsalzlösung verabreicht.

Gruppen von jeweils 6 Tieren, die am Tag 1 mit Monocrotalin behandelt wurden, wurden an den Versuchtagen 14-28 täglich entweder 0.25 mg/kg Lisurid oder 2.5 mg/kg Tergurid per Schlundsonde verabreicht. Die Dosisangaben beziehen sich hierbei auf die freie Base der Substanzen. Die Substanzen wurden als Hydrogenmaleat-Salz bzw. freie Base eingesetzt. Sie wurden in Gegenwart von Spuren von Ascorbinsäure in destilliertem Wasser aufgenommen und in einem Volumen von 2 mL per Schlundsonde Morgens und Abends verabreicht. Kontrolltieren wurde die gleiche Menge Wasser verabreicht.

Am Versuchstag 28 wurden 2 Stunden nach der letzten Substanzgabe die Tiere mit Pentobarbital in Narkose versetzt. Anschließend wurden an den Tiere eine Tracheostomie ausgeführt und die Tiere mit 10 ml/kg und einer Frequenz von 60s⁻¹ beatmet (SAR830A/P; IITC). Die Narkose wurde durch Inhalation von Isofluran aufrechterhalten.

Der mittlere arterielle Druck sowie der rechte ventrikuläre systolische Blutdruck wurden bestimmt. Der systemische arterielle Druck wurde durch einen Millarkatheter in der linken Karotis Arterie gemessen. Durch die rechte Jugularvene wurde ein Millar Katheter mit einem Drucksensor (Millar Instruments, Modell. SPR-534) eingeführt und bis in den rechten Ventrikel des Herzens geschoben und zur Messungen des rechten Ventrikeldrucks (RVSP) eingesetzt. Das Signal wurde mittels eines HSE Couplers Series 500 verstärkt und einer Registriereinheit zur Auswertung zugeführt.

Nach Durchführung der Druckmessungen wurden die Ratten mit physiologischer Kochsalzlösung perfundiert. Der rechte Lungenflügel wurde entnommen, tiefgefroren und zur Bestimmung des Collagengehalts aufgearbeitet. Hierzu wurde das Gewebe zunächst homogenisiert und in Anlehnung an die Methode von Berg (Meths Enzymol. 82, 372 (1982)) bestimmt. Zunächst wurde eine Hydrolyse der Probe in 6 molarer Salzsäure für 16 Stunden bei 116°C durchgeführt. Nachgeschaltet erfolgte eine Oxidation von Hydroxyprolin zu Pyrrol, gefolgt von einer Komplexbildung mit p-Dimethylaminobenzaldehyd. Der entstandene Farbkomplex wurde bei 560 nm photometrisch vermessen und mittels einer Eichkurve wurde der Hydroxyprolin-Gehalt der Proben ermittelt. Die Ergebnisse sind als µg/g Protein im Lungengewebe angegeben.

### Ergebnisse:

a) Einfluß der Behandlung mit Lisurid oder Tergurid an den Versuchstagen 15-28 auf den systolischen Druck im rechten Herzventrikel (RVPsys) und den systemischen arteriellen Druck (SAP)

| | RVPsys [mm Hg] | SAP [mm Hg] |
|---|---|---|
| Kontrolle | 23± 4 | 118±5 |
| Monocrotalin | 55± 5 | 114±7 |
| Monocrotalin + 0,25mg/kg Lisurid bid | 43±7 | 109±9 |
| Monocrotalin +2,5 mg/kg Terqurid bid | 39±3 | 111±7 |

Ergebnisse sind Mittelwerte±SEM (N=6)
b) Einfluß der Behandlung mit Lisurid oder Tergurid an den Versuchstagen 15-28 auf den Hydroxyprolin-Gehalt der Lunge

| | Hydroxyprolin [ug/g Protein] |
|---|---|
| Kontrolle | 1,2 ± 0,2 |
| Monocrotalin | 4,2 ± 1,1 |
| Monocrotalin + 0.25mg/kg Lisurid bid | 3,3 ± 0.8 |
| Monocrotalin +2,5 mg/kg Tergurid bid | 2,7 ± 1.2 |

Ergebnsse sind Mittelwerte ±SEM (N=6)

### Ergebnisbewertung:

Nach Verabreichung von Monocrotalin kommt es in Ratten zu einer Endothelschädigung der Lunge, die mit einer überschießenden Produktion von Bindegewebe und der Entwicklung von Lungenhochdruck verbunden sind. Die Akkumulation von Collagen, gemessen als Hydroxyprolin-Gehalt im Lungengewebe sowie der systolische Druckanstieg in der rechten Herzkammer reflektieren diese strukturellen und funktionellen Veränderungen. In diesem Modell für Lungenhochdruck wurden mögliche therapeutische Wirkungen einer Behandlung mit Lisurid oder Tergurid geprüft. Die Therapie setzte unter den experimentellen Bedingungen nicht zum Zeitpunkt der Monocrotalin Behandlung, d.h. nicht zum Zeitpunkt der Schädigung, sondern erst nach 14 Tagen ein. Zu diesem Zeitpunkt haben sich nach Literaturangaben umfangreiche Gefäßveränderungen und ein Druckanstieg manifestiert. Die Therapie mit Lisurid oder Tergurid vermindert den Druckanstieg in der rechten Herzkammer als ein indirektes Maß für den Lungenhochdruck im Sinne eines therapeutisch gewünschten Effektes.

Als strukturelles Korrelat wurde unter Therapie mit beiden Substanzen eine Verminderung des durch Monocrotalin erhöhten Hydroxyprolin-Gehaltes im Sinne eines "reversen Remodellings" beobachtet. Lisurid und Tergurid weisen in diesem etablierten Tiermodell Wirkqualitäten auf, die einen therapeutischen Einsatz an Patienten mit Lungenhochdruck erfolgreich machen.

Das beschriebene Versuchsbeispiel belegt die erfolgreiche Verwendung der Ergoline am Beispiel von Lisurid und Tergurid zur Behandlung von Lungenhochdruck.

### Beispiel 9: Pulmonaler Hochdruck:

### Versuchsbeschreibung:

Ratten wurden am Versuchstag 1 Monocrotalin (60 mg/kg; Sigma) verabreicht. Die Substanz wurde hierzu in 0.5 molarer Salzsäure gelöst und anschliessend der pH mit 0.5 molarer Natronlauge auf 7.4 adjustiert. Die Lösung wurde als einmalige subkutane Injektion in einer Dosis von 60 mg/kg an männliche Sprague Dawley Ratten verabreicht. Kontrolltieren wurde das gleiche Volumen isotonischer Kochsalzlösung verabreicht.

### Induktion von pulmonalem arteriellem Hochdruck

Gruppen von jeweils 4 Tieren, die am Tag 1 mit Monocrotalin behandelt wurden, wurden an den Versuchtagen 1-28 zweimal täglich 1.2 mg/kg Tergurid intraperitoneal verabreicht. Tergurid wurde in Gegenwart von Spuren von Ascorbinsäure in destilliertem Wasser aufgenommen und in einem Volumen von 2 mL per Schlundsonde Morgens und Abends verabreicht. Kontrolltieren wurde die gleiche Menge physiologischer Kochsalzlösung verabreicht.

Am Versuchstag 28 wurden 2 Stunden nach der letzten Substanzgabe die Tiere mit Pentobarbital in Narkose versetzt. Anschließend wurden an den Tieren eine Tracheostomie ausgeführt und die Tiere mit 10 ml/kg und einer Frequenz von 60s⁻¹ beatmet (SAR830A/P; IITC). Die Narkose wurde durch Inhalation von Isofluran aufrechterhalten.

### Bestimmung von Rechtsventrikulärem systolischem Druck (RVSP) und SAP (Systolischem Arteriellem Druck)

Der mittlere arterielle Druck sowie der rechte ventrikuläre systolische Blutdruck wurden bestimmt. Der systemische arterielle Druck wurde durch einen Millarkatheter in der linken Karotis Arterie gemessen. Durch die rechte Jugularvene wurde ein Millar Katheter mit einem Drucksensor (Millar Instruments, Modell. SPR-534) eingeführt und bis in den rechten Ventrikel des Herzens geschoben und zur Messungen des rechten Ventrikeldrucks (RVSP) eingesetzt. Das Signal wurde mittels eines HSE Couplers Series 500 verstärkt und einer Registriereinheit zur Auswertung zugeführt.

### Bestimmung der Rechtsherzhypertrophie

Nach Durchführung der Druckmessungen wurden die Ratten mit physiologischer Kochsalzlösung perfundiert. Das Herz wurde entnommen sowie durch Dissektion der rechte Ventrikel, das Septum sowie der linke Ventrikel jedes Herzens präpariert. Die Gewebepräparationen wurden gefriergetrocknet und anschließend die Trockengewichte durch Auswiegen bestimmt. Aus diesen Tieren wurde für jedes individuelle Tier der Quotient: Gewicht des rechten Ventrikels/ Gewicht von linken Ventrikel und Septum (RV/LV+S) als ein Maß der Rechtsherzhypertrophie bestimmt.

### Bestimmung der Muskularisierung von arteriellen Kapillargefäßen der Lunge

Die Lungen wurden entnommen, in Formalin fixiert und in Paraffin eingebettet. 3 um Paraffin Schnitte wurden gewonnen und eine immunhistochemische Doppelfärbung nach Standardprotokollen durchgeführt. Glatte Muskelzellen der Gefäße wurden mit einem Antikörper gegen Aktin und das Endothel mittel Antikörper gegen von Willebrand Faktor angefärbt. Für die Auswertung wurden ≥ 80 intraazinäre Arterien mit einem Durchmesser > 50 µm herangezogen. Die Gefäße wurden in 3 Kategorien unterteilt: nicht-muskularisierte Gefäße mit < 20 %, teilweise muskularisierte Gefässe mit >20 %, aber < 70%, und voll muskularisierte Gefäße mit >70 % Umkleidung des Gefäßquerschnitts mit glatten Muskelzellen.

### Ergebnisse:

a) Einfluß der Behandlung mit Tergurid an den Versuchstagen 1-28 auf den systolischen Druck im rechten Herzventrikel (RVSP) und den systemischen arteriellen Druck (SAP)

| | RVSP [mm Hg] | SAP [mm Hg] |
|---|---|---|
| Kontrolle | 31,0 ± 3,6 | 104,0 ± 13,3 |
| Monocrotalin | 64,4 ± 14,5 | 93,7 ± 19,4 |
| Monocrotalin + 0,4mg/kg Terguride bid | 36,4 ± 3,6 | 97,5 ± 15,2 |

Ergebnisse sind Mittelwerte ± SD (N=4)
b) Einfluß der Behandlung mit Tergurid an den Versuchstagen 1-28 auf die Rechtsherzhypertrophie, gemessen als Verhältnis: Gewicht von rechtem Herzventrikel vs. Gewicht von linkem Herzventrikel und Septum.

| | RV/LV+S |
|---|---|
| Kontrolle | 0,31 ± 0,06 |
| Monocrotalin | 0,74 ± 0,14 |
| Monocrotalin + 0,4mg/kg Terguride bid | 0,33 ± 0,08 |

Ergebnisse sind Mittelwerte ± SD (N=4)
c) Einfluß der Behandlung mit Tergurid an den Versuchstagen 1-28 auf die Muskularisierung von arteriellen Kapillargefäßen (20-50 µm Durchmesser) in der Lunge

| | Anteil nichtmuskularisierter Gefäße [%] | Anteil teilmuskularisierter Gefäße [%] | Anteil voll muskularisierter Gefäße [%] |
|---|---|---|---|
| Kontrolle | 57,8 ± 14,6 | 3,2 ± 2,3 | 14,8 ± 6,2 |
| Monocrotalin | 40,4 ± 16,0 | 42,3 ± 4,4 | 64,0 ± 7,7 |
| Monocrotalin + 0,4mg/kq Terquride bid | 1,9 ± 1,6 | 57,8 ± 14,6 | 21,3 ± 12,6 |

Ergebnisse sind Mittelwerte ± SD (N=4)

### Ergebnisbewertung:

Nach Verabreichung von Monocrotalin kommt es in Ratten zu einer Endothelschädigung der Lunge, die mit einer überschießenden Proliferation von glatten Muskelzellen in arteriellen Kapillargefäßen der Lunge und der Entwicklung von Lungenhochdruck verbunden sind. Der systolische Druckanstieg in der rechten Herzkammer bei konstantem systemischem Blutdruck sowie die Rechtsherzhypertrophie reflektieren diese strukturellen und funktionellen Veränderungen.

In diesem Modell für Lungenhochdruck wurden mögliche therapeutische Wirkungen einer Behandlung mit Tergurid geprüft. Die Therapie setzte unter den experimentellen Bedingungen unmittelbar nach dem Zeitpunkt der Monocrotalin Gabe ein. Unter Therapie mit Tergurid wird der Druckanstieg in der rechten Herzkammer als ein indirektes Maß für den Lungenhochdruck nachzuständig unterdrückt. Der systemische Blutdruck ist unter den Versuchsbedingungen nicht nachweisbar verändert.

Ebenso kommt es unter Therapie zu einer vollständigen Unterdrückung der Monocrotalin induzierten Rechtsherzhypertrophie. In der Lunge wurde unter Therapie als therapeutische Wirkung eine Verminderung der Monocrotalininduzierten Muskularisierung von arteriellen Kapillargefäßen beobachtet. Tergurid weist in diesem etablierten Tiermodell für pulmonalen Hochdruck Wirkqualitäten in allen relevanten funktionellen und strukturellen Parametern auf, die einen therapeutischen Einsatz an Patienten mit Lungenhochdruck erfolgreich machen.

### Beispiel 10: Hemmung der Expression con Col IA2 in glatten Muskelzellen der Lungenarterien

In dem Experiment wurde ein möglicher stimulierender Einfluss von Serotonin auf die Expression von Col1 A2 sowie die Hemmwirkung von Tergurid untersucht. Die Untersuchungen wurden an humane pulmonale glatte Muskelzellen (Cambrex) durchgeführt.. Die Zellen wurden nach Herstellerangaben in CC-3182 Medium (Cambrex) kultiviert. 5% eines mit Aktivkohle vorbehandelten Serotonin depletierten fötalen Kälberserums (FCS) (HyClone) wurden zugesetzt.

Nach Erreichen eines konfluenten Zellrasens wurden die Zellen für weitere 2 Tage in 5% FCS kultiviert. Für das Experiment wurde ein Medium mit Zusatz von 0.5% FCS eingesetzt. Nach Zugabe von Serotonin (100 nmol/L) und/oder Terguride (100 nmol/L) wurden die Zellen für 48 Stunden weiterkultiviert. Die Inkubationen wurden in Triplikaten durchgeführt.

Gesamt RNS wurde mit dem RNeas Kit (Qiagen) gemäß Herstellerangaben gewonnen. Die reverser Transcription in cDNA erfolgte mittel oligo-dT Primer (Roche).

Anschließend wurde die Genexpression mittels SYBR Green real-time PCR an einem ABI Prism 7700 Sequence Detection System (Applied Biosystems, Foster City, CA, USA) nach Standardprotokollen quantifiziert. Als spezifisches Primer-Paar für humanes Col1 A2 wurden in die PCR-Analyse der Vorwärts Primer: 5'-GGTCAGCACCA-CCGATGTC-3', und der reverse Primer: 5'-CACGCCTG-CCCTTCCTT-3' eingesetzt.

Zur Standardisierung von Unterschieden in der Gesamtmenge an RNA in einzelnen Proben, wurde die Expression von Col1 A2 normiert auf die Expression des Enzyms Glycerinaldehyd-3-Phosphat Dehydrogenase (GAPDH), welches in den Zellen konstitutiv exprimiert wird. Hierzu wurden folgende Primer benutzt: Vorwärts Primer: 5'-CAATGC-CTCCTGCACCACCAAC-3'und reverser Primer: 5'-AGGGGCCATCCACAGTCTTCT-3'.

Die Expressionsleistung von Col1 A2 und GAPDH in individuellen Proben wurde mit Standardauswertungsmethoden bestimmt und als Col1A2/GAPDH Verhältnisse quantifiziert.

Die Darstellung der Ergebnisse erfolgte als "box-and whisker" Plots.

Wie aus der Abbildung zu ersehen ist, wird in human glatten Musklezellen aus Pulmonalarterien die Expression von Col1 A2 in Gegenwart von 100 nmol/L Serotonin im Vergleich zu einem Kontrollansatz deutlich stimuliert. Dies deutet auf eine trophischen Wirkung von Serotonin hin. Eine überschiessende Deposition von Collagen leistet zusammen mit der Proliferation von glatten Muskelzellen einen Beitrag zur Pathophysiologie von pulmonalem arteriellem Hochdruck.

In Gegenwart von Terguride wird die Expression von Col1 A2 gehemmt, wobei die Hemmwirkung in Gegenwart von Serotonin stärker ausgeprägt ist. Aus der hier beschriebenen Wirkqualität lässt sich deshalb ein möglicher therapeutischer Nutzen von Tergurid für die Anwendung bei Patienten mit pulmonalem Hochdruck ableiten.

## Patentansprüche

1. Lisurid oder Tergurid sowie Salze, Enantiomere, Enantiomerengemische, Diastereomere, Diastereomerengemische, Hydrate, Solvate und Racemate davon zur Verwendung bei der Behandlung und Prophylaxe von pulmonalem arteriellem Hochdruck, endogen-induzierter oder exogen-induzierter Glomerulosklerosen und sekundärem Raynaud Syndrom.

2. Verwendung von Lisurid oder Tergurid sowie Salzen, Enantiomeren, Enantiomerengemischen, Diastereomeren, Diastereomerengemischen, Hydraten, Solvaten und Racematen davon zur Herstellung einer pharmazeutischen Zusammensetzung zur Behandlung und Prophylaxe von pulmonalem arteriellem Hochdruck, endogen-induzierter oder exogen-induzierter Glomerulosklerosen und sekundärem Raynaud-Syndrom.

3. Pharmazeutische Zusammensetzung enthaltend mindestens eine der Verbindungen Lisurid oder Tergurid sowie Salze, Enantiomere, Enantiomerengemische, Diastereomere, Diastereomerengemische, Hydrate, Solvate und Racemate davon in einer aktiven Wirkstoffkonzentration von 0,1 - 10 mg pro Einzeldosis zusammen mit pharmakologisch verträglichen Trägern, Hilfsstoffen und/oder Lösungsmitteln zur Verwendung bei der Behandlung und Prophylaxe von pulmonalem arteriellem Hochdruck, endogen-induzierter oder exogen-induzierter Glomerulosklerosen und sekundärem Raynaud Syndrom.

4. Pharmazeutische Zusammensetzung zur Verwendung gemäß Anspruch 3, wobei die pharmazeutische Zusammensetzung zur oralen, sublingualen, parenteralen, kutanen, bukkalen, perkutanen, subcutanen, inhalativen oder nasalen Verabreichung geeignet ist.

5. Pharmazeutische Zusammensetzung zur Verwendung gemäß Anspruch 3 oder 4 in Form von Tabletten, Schichttabletten, Kapseln, Retard-Oralia, Suppositorien, Mikroformulierungen, Nanoformulierungen, liposomalen Formulierungen, Tropfen, Nasentropfen, Nasensprays, Aerosolen, Ampullen, Lösungen, Emulsionen, Dispersionen, Pulvern, Inhalationspulvern, mikrokristallinen Formulierungen, Inhalationssprays, transdermalen Systemen oder subcutanen Formulierungen.

## Claims

1. Lisuride or terguride as well as salts, enantiomers, mixtures of enantiomers, diastereomers, mixtures of diastereomers, hydrates, solvates and racemates thereof for use in the treatment and prophylaxis of pulmonary arterial hypertension, endogenously induced or exogenously induced glomeruloscleroses and secondary Raynaud's syndrome.

2. Use of lisuride or terguride as well as salts, enantiomers, mixtures of enantiomers, diastereomers, mixtures of diastereomers, hydrates, solvates and racemates thereof for preparation of a pharmaceutical composition for the treatment and prophylaxis of pulmonary arterial hypertension, endogenously induced or exogenously induced glomeruloscleroses and secondary Raynaud's syndrome.

3. A pharmaceutical composition, comprising at least one of the compounds lisuride or terguride as well as salts, enantiomers, mixtures of enantiomers, diastereomers, mixtures of diastereomers, hydrates, solvates and racemates thereof in a concentration of active substance of 0.1-10 mg per single dose along with pharmacologically acceptable carriers, auxiliary substances and/or solvents for use in the treatment and prophylaxis of pulmonary arterial hypertension, endogenously induced or exogenously induced glomeruloscleroses and secondary Raynaud's syndrome.

4. A pharmaceutical composition for use according to Claim 3, wherein the pharmaceutical composition is suitable for oral, sublingual, parenteral, cutaneous, buccal, percutaneous, subcutaneous, inhalative or nasal administration.

5. A pharmaceutical composition for use according to Claim 3 or 4 in the form of tablets, layered tablets, capsules, Retard-Oralia, suppositories, microformulations, nanoformulations, liposomal formulations, drops, nose drops, nasal sprays, aerosols, ampoules, solutions, emulsions, dispersions, powders, inhalation powders, microcrystalline formulations, inhalation sprays, transdermal systems or subcutaneous formulations.

## Revendications

1. Lisuride ou terguride ainsi que sels, énantiomères, mélanges d'énantiomères, diastéréomères, mélanges de diastéréomères, hydrates, solvates et racémiques de ces dernières pour utlisation dans le traitement et la prophylaxie de l'hypertonie artérielle pulmonaire, des gloméruloscléroses induites par voie endogène ou induites par voie exogène et du syndrome de Raynaud secondaire.

2. Utilisation de la lisuride ou terguride ainsi que de sels, énantiomères, mélanges d'énantiomères, diastéréomères, mélanges de diastéréomères, hydrates, solvates et racémiques de ces dernières pour la préparation d'une composition pharmaceutique destinée au traitement et à la prophylaxie de l'hypertonie artérielle pulmonaire, de gloméruloscléroses induites par voie endogène ou induites par voie exogène et du syndrome de Raynaud secondaire.

3. Composition pharmaceutique contenant au moins un des composés lisuride ou terguride ainsi que sels, énantiomères, mélanges d'énantiomères, diastéréomères, mélanges de diastéréomères, hydrates, solvates et racémiques de ces dernières dans une concentration de substances active de 0,1-10 mg par dose individuelle ensemble avec des véhicules, substances auxiliaires et/ou solvants pharmacologiquement acceptables pour utiisation dans le traitement et la prophylaxie de l'hypertonie artérielle pulmonaire, des gloméruloscléroses induites par voie endogène ou induites par voie exogène et du syndrome de Raynaud secondaire.

4. Composition pharmaceutique pour utilisation selon la revendication 3, étant donné que la composition pharmaceutique est appropriée pour l'administration orale, sous-linguale, parentérale, cutanée, buccale, percutanée, sous-cutanée, par inhalation ou nasale.

5. Composition pharmaceutique pour utilisation selon la revendication 3 ou 4 sous forme de comprimés, comprimés stratifiés, capsules, Retard-Oralia, suppositoires, microformulations, nanoformulations, formulations liposomales, gouttes, gouttes nasales, sprays nasals, aérosols, ampoules, solutions, émulsions, dispersions, poudres, poudres à inhaler, formulations microcristallines, sprays à inhaler, systèmes transdermiques ou formulations sous-cutanées.
